# EUROPÄISCHE PATENTSCHRIFT

(11) **EP 1 129 062 B1**
(45) Veröffentlichungstag und Bekanntmachung des Hinweises auf die Patenterteilung: **28.04.2004**
(21) Anmeldenummer: 99959272.8
(22) Anmeldetag: 05.11.1999
(51) Int. Cl.: C07C 67/60, C07C 69/54, C07C 67/08

(54) **VERFAHREN ZUM VERESTERN VON (METH)ACRYLSÄURE MIT EINEM ALKANOL**
METHOD FOR ESTERIFYING (METH)ACRYLIC ACID WITH AN ALKANOL
PROCEDE D'ESTERIFICATION D'ACIDE (METH)ACRYLIQUE AVEC UN ALCANOL

(30) Priorität: 11.11.1998 DE 19851984
(43) Veröffentlichungstag der Anmeldung: 05.09.2001
(73) Patentinhaber: BASF AKTIENGESELLSCHAFT, 67056 Ludwigshafen (DE)
(72) Erfinder: AICHINGER, Heinrich, D-68199 Mannheim (DE); HERBST, Holger, D-67227 Frankenthal (DE); NESTLER, Gerhard, D-67061 Ludwigshafen (DE); SCHRÖDER, Jürgen, D-67071 Ludwigshafen (DE); BECKMANN, Stefan, D-67098 Bad Dürkheim (DE)
(86) Internationale Anmeldenummer: PCT/EP1999/008503
(87) Internationale Veröffentlichungsnummer: WO 2000/027789

(56) Entgegenhaltungen:
- DE-A- 19 547 485
- DE-A- 19 701 737

## Beschreibung

Vorliegende Erfindung betrifft ein Verfahren zum Verestern von (Meth)acrylsäure mit einem Alkanol in Gegenwart eines Veresterungskatalysators, bei dem man vom Reaktionsgemisch nicht umgesetzte Ausgangsverbindungen und gebildeten (Meth)acrylsäureester unter Verbleib eines Oxyester enthaltenden Sumpfproduktes destillativ entfernt, das Sumpfprodukt abtrennt und nach der Abtrennung des Sumpfproduktes die in selbigem enthaltenen Oxyester im Beisein wenigstens eines Säurekatalysators durch Einwirkung erhöhter Temperatur spaltet und die Spaltung stufenförmig durchgeführt wird.

Der Begriff "(Meth)acrylsäure" steht in dieser Anmeldung verkürzend für Acryl- oder Methacrylsäure.

Ferner meint die im weiteren Verlauf dieser Anmeldung verwendete Bezeichnung "oligomere (Meth)acrylsäure" die Michael-Addukte von (Meth)acrylsäure mit sich selbst und den dabei entstehenden Folgeprodukten. Solche Michael-Addukte lassen sich durch die allgemeine Formel (III), 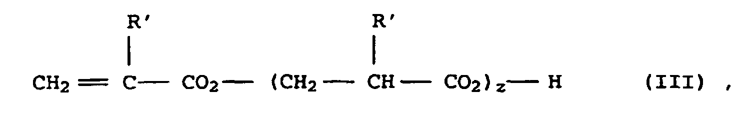 mit z = eine ganze Zahl von 1 bis 5
und R' = H oder CH₃,
charakterisieren und sind hier von (monomerer) (Meth)acrylsäure sowie von (Meth)acrylsäurepolymerisaten (die durch radikalische Polymerisation von (Meth)acrylsäure erhältlich sind) zu differenzieren. Wesentlich ist, daß die Michael-Addition von (Meth)acrylsäure mit sich selbst und dabei entstehenden Folgeprodukten reversibel ist. Oligomere (Meth)acrylsäure fällt z.B. bei der destillativen Behandlung von (z.B. roher) (Meth)acrylsäure (der Begriff "roh" weist einen noch enthaltenen geringen Anteil an insbesondere aldehydischen Verunreinigungen aus) im Sumpf an (vgl. z.B. DE-A 22 35 326).

Üblicherweise erfolgt die Herstellung von Alkylestern der (Meth)acrylsäure durch Veresterung von (Meth)acrylsäure mit Alkanolen bei erhöhter Temperatur in flüssiger Phase mit oder ohne Lösungsmittel und in Gegenwart von von (Meth)acrylsäure verschiedenen Säuren als Katalysator (vgl. z.B. DE-A 23 39 519). Nachteilig an dieser Herstellungsweise ist, daß sich unter den vorgenannten Veresterungsbedingungen als Nebenreaktionen noch nicht umgesetzter Ausgangsalkohol unter Ausbildung einer Verbindung der nachfolgend angeführten allgemeinen Formel I sowie noch nicht umgesetzte (Meth)acrylsäure unter Ausbildung einer Verbindung der allgemeinen Formel II an die ethylenisch ungesättigte Doppelbindung von bereits gebildetem (Meth)acrylsäurealkylester addiert (Michael-Addition).

Auch eine sukzessive Mehrfachaddition ist möglich. Ferner können gemischte Typen auftreten. Diese Addukte (Alkoxyester und Acyloxyester) nennt man kurz Oxyester: 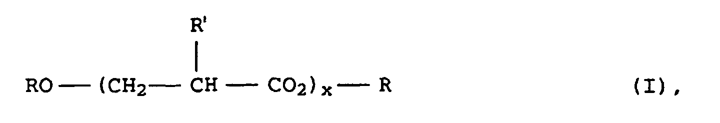 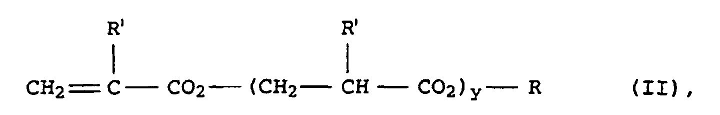 mit x,y = eine ganze Zahl von 1 bis 5,
R = Alkyl und
R' = H oder CH₃.

Bei der Herstellung von Estern der Acrylsäure ist das Problem der Oxyesterbildung besonders ausgeprägt, wobei die hauptsächlich gebildeten Oxyester der Alkoxypropionsäureester und der Acyloxypropionsäureester mit x, y = 1 sind. Bei der Herstellung von Estern der Methacrylsäure erfolgt die Oxyesterbildung in geringerem Maße. Die Entstehung von Oxyestern ist u.a. in der DE-A 23 39 529 sowie in der US-A 5 734 075 beschrieben. Vorstehende Schriften bestätigen, daß die Bildung von Oxyestern im wesentlichen unabhängig von den-speziellen Veresterungsbedingungen erfolgt. Von ganz besonderer Bedeutung ist die Oxyesterbildung bei der Herstellung von Acrylsäureestern der C₁- bis C₈-Alkanole, insbesondere der C₄- bis C₈-Alkanole, ganz besonders bei der Herstellung von n-Butylacrylat und 2-Ethylhexylacrylat (weshalb vorliegende Erfindung insbesondere bezüglich dieser Veresterungen Anwendung findet).

Charakteristisch für die Oxyester ist, daß ihr Siedepunkt oberhalb der Siedepunkte von Ausgangssäure, Ausgangsalkohol, gebildeten Zielester, sowie gegebenenfalls mitverwendetem organischem Lösungsmittel liegt.

Die Aufarbeitung eines beliebigen Veresterungs-Reaktionsgemisches erfolgt normalerweise so, daß nicht umgesetzte Ausgangsverbindungen sowie gebildeter Zielester vom Reaktionsgemisch destillativ abgetrennt werden, wobei der zur Veresterung verwendete Säurekatalysator gegebenenfalls vorher durch Extraktion mittels Wasser und/oder wäßriger Lauge abgetrennt wird (vgl. z.B. Ullmann's Encyclopedia of Industrial Chemistry, Vol. A1, 5th Ed., VCH S. 167ff). Das im Rahmen einer solchen destillativen Aufarbeitung verbleibende Sumpfprodukt enthält die Oxyester, die einen beträchtlichen Ausbeuteverlust bedingen.

Der Stand der Technik (z.B. DE-A 19 701 737, DE-A 19 536 191, DE-A 19 536 184, DE-A 19 547 485, DE-A 19 547 459 und CN-A 1063678) enthält daher verschiedene Verfahren, die die im abgetrennten Sumpfprodukt enthaltenen Oxyester im Beisein wenigstens eines Säurekatalysators durch Einwirkung erhöhter Temperatur rückspalten und die dabei entstehenden Spaltprodukte, vorzugsweise durch Abdampfen, abtrennen. Als geeignete saure Spaltkatalysatoren werden dabei von monomerer und oligomerer (Meth)acrylsäure verschiedene, protische Säuren (Säurestärke > als diejenige von (Meth)acrylsäure) wie z.B. Mineralsäuren wie Schwefelsäure oder Phosphorsäure sowie organische Säuren wie Methansulfonsäure oder p-Toluolsulfonsäure empfohlen. Dieser Begriffsinhalt gilt auch für diese Anmeldung.

Nachteilig an den Rückspaltverfahren des Standes der Technik ist jedoch, daß der sich bildende Spaltrückstand meist hochviskos und in der Regel feststoffhaltig anfällt.

In der Folge ist der Spaltrückstand kaum pumpfähig und deshalb nur schwer einer Entsorgung zuzuführen. Außerdem scheidet sich der meist teerartige Feststoff im Verlauf der Zeit an den Wandoberflächen ab (Fouling), was z.B. den Wärmedurchgang mindert oder zu Verstopfungen führen kann, weshalb die Wandoberflächen von Zeit zu Zeit gesäubert werden müssen.

Die US-A 5 734 075 empfiehlt zur Minderung der vorgenannten Problematik, das Rückspaltverfahren in Abwesenheit von sauren Spaltkatalysatoren und statt dessen im Beisein von oligomerer (Meth)acrylsäure durchzuführen. Nachteilig an dieser Verfahrensweise ist jedoch, daß die Rückspaltung, insbesondere im Fall von Sumpfprodukten, die von Veresterungen mit längerkettigen Alkanolen herrühren, vergleichsweise langsam abläuft und nur vergleichsweise geringe Umsätze liefert. Die DE-A 19 536 184 empfiehlt zur Minderung der genannten Problematik die Oxyester vor deren Rückspaltung vom Sumpfprodukt destillativ abzutrennen. Nachteilig an dieser Verfahrensweise ist das Erfordernis eines zusätzlichen Destillationsschrittes.

Es wurde auch schon vorgeschlagen zur Minderung der Viskositätsproblematik ein Lösungsmittel als Verdünnungsmittel zuzusetzen. Der Bedarf einer zusätzlichen Komponente ist jedoch gleichfalls nachteilig.

Aufgabe der vorliegenden Erfindung war es daher, ein Verfahren zum Verestern von (Meth)acrylsäure mit einem Alkanol in Gegenwart eines Veresterungskatalysators, bei dem man vom Reaktionsgemisch nicht umgesetzte Ausgangsverbindungen und gebildeten (Meth)acrylsäureester unter Verbleib eines Oxyester enthaltenden Sumpfproduktes destillativ entfernt, das Sumpfprodukt abtrennt und nach der Abtrennung des Sumpfproduktes die in selbigem enthaltenen Oxyester im Beisein wenigstens eines Säurekatalysators durch Einwirkung erhöhter Temperatur spaltet und die Spaltung stufenförmig durchgeführt wird, zur Verfügung zu stellen, das die genannten Nachteile der Verfahren des Standes der Technik nicht aufweist.

Demgemäß wurde ein Verfahren zum Verestern von (Meth)acrylsäure mit einem Alkanol in Gegenwart eines Veresterungskatalysators, bei dem man vom Reaktionsgemisch nicht umgesetzte Ausgangsverbindungen und gebildeten (Meth)acrylsäureester unter Verbleib eines Oxyester enthaltenden Sumpfproduktes destillativ entfernt, das Sumpfprodukt abtrennt und nach der Abtrennung des Sumpfproduktes die in selbigem enthaltenen Oxyester im Beisein wenigstens eines Säurekatalysators, wobei als wenigstens ein Säurekatalysator zur Spaltung der Oxyester wenigstens eine aromatische Sulfonsäure der allgemeinen Formel IV, 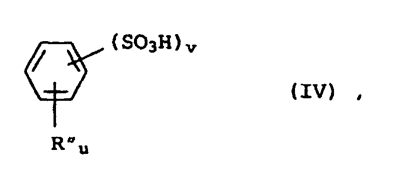 mit
- R" =: unabhängig voneinander ein sechs bis zwanzig C-Atome aufweisender Alkylrest,
- u =: eine ganze Zahl von 1 bis 3,
- v =: 1 oder 2,
mitverwendet wird, durch Einwirkung erhöhter Temperatur spaltet, dadurch gekennzeichnet, daß der Anteil der aromatischen Sulfonsäuren der allgemeinen Formel (IV), bezogen auf die Gesamtmenge der zur Spaltung der Oxyester eingesetzten Säurekatalysatoren, wenigstens 50 mol-% beträgt, und die Spaltung stufenförmig durchgeführt wird.

Vorgenannte Sulfonsäuren der allgemeinen Formel (IV) sind z.B. aus der EP-A 521 488 bekannt. u kann sowohl 1 oder 2 oder 3 betragen, wobei v gleichzeitig 1 oder 2 sein kann. Häufig sind die Reste R" acht bis sechzehn bzw. zehn bis vierzehn C-Atome aufweisende Alkylreste. Geeignete Vertreter von Verbindungen (IV) sind demnach z.B. Octylbenzolsulfonsäuren wie n-Octylbenzolsulfonsäure, Nonylbenzolsulfonsäuren wie n-Nonylbenzolsulfonsäure, Decylbenzolsulfonsäuren wie n-Decylbenzolsulfonsäure, Undecylbenzolsulfonsäuren wie n-Undecylbenzolsulfonsäure, Dodecylbenzolsulfonsäuren wie n-Dodecylbenzolsulfonsäure, Tridecylbenzolsulfonsäuren wie n-Tridecylbenzolsulfonsäure, Tetradecylbenzolsulfonsäuren wie n-Tetradecylbenzolsulfonsäure, Pentadecylbenzolsulfonsäuren wie n-Pentadecylbenzolsulfonsäure, Hexadecylbenzolsulfonsäuren wie n-Hexadecylbenzolsulfonsäure, Heptadecylbenzolsulfonsäuren wie n-Heptadecylbenzolsulfonsäure, Octadecylbenzolsulfonsäuren wie n-Octadecylbenzolsulfonsäure, Nonyldecylbenzolsulfonsäuren wie n-Nonyldecylbenzolsulfonsäure, Heptadecylbenzolsulfonsäuren wie n-Heptadecylbenzolsulfonsäure, Octadecylbenzolsulfonsäuren wie n-Octadecylbenzolsulfonsäure, Nonyldecylbenzolsulfonsäuren wie n-Nonyldecylbenzolsulfonsäure und Eicosylbenzolsulfonsäuren wie n-Eicosylbenzolsulfonsäure. Selbstverständlich können erfindungsgemäß auch Gemische von Verbindungen (IV) verwendet werden. Derartige Gemische werden in der Regel dann eingesetzt, wenn man Verbindungen (IV) verwendet, die lediglich technische Reinheit aufweisen. Beispiele für derartige technische, im Handel käuflich erwerbliche, Verbindungen (IV) sind z.B. die Alkylbenzolsulfonsäuren Bio-Soft® S-100 (mittleres Molekulargewicht ca. 318, mittlere R" Kettenlänge = 11,5 C-Atome; Hersteller = Stepan Co.), AAS-985 (lineare Alkylbenzolsulfonsäure mit einer mittleren Alkylkettenlänge von C₁₁-C₁₂, Hersteller = Continental Chemical Co.), Vista SA 697 und Vista SA 597 (lineare Alkylbenzolsulfonsäuren mit einem mittleren Molekulargewicht von 342 bzw. 318, Hersteller = Vista Chemical Co.), Stepantan® H-100 (eine verzweigte Dodecylbenzolsulfonsäure, Hersteller = Stepan Co.) sowie eine technische Alkylbenzolsulfonsäure der Fa. Alfa Products Co., bei der R" zu 1 Gew.-% aus C₁₀, zu 40 Gew.-% aus C₁₁, zu 28 Gew.-% aus C₁₂ und zu 31 Gew.-% aus C₁₃ besteht.

Die erfindungsgemäß zu verwendenden Sulfonsäuren (IV) können im Rahmen des erfindungsgemäßen Verfahrens sowohl als alleinige saure Spaltkatalysatoren als auch im Gemisch mit den im zitierten Stand der Technik empfohlenen sauren Spaltkatalysatoren (z.B. Schwefelsäure. Phosphorsäure, Methansulfonsäure und/oder p-Toluolsulfonsäure) angewendet werden. D.h., bezogen auf die beim erfindungsgemäßen Verfahren insgesamt eingesetzte Menge an sauren Spaltkatalysatoren kann der molare Anteil der erfindungsgemäßen Verbindungen (IV) z.B. ≥ 50 mol-%, ≥ 75 mol-%, ≥ 90 mol-%, ≥ 95 mol-% oder aber auch 100 mol-% betragen. Vorzugsweise beträgt der vorgenannte Anteil der erfindungsgemäßen Verbindungen (IV) wenigstens 50 mol-%, besonders bevorzugt wenigstens 75 mol-% und mit besonderem Vorteil 100 mol-%.

In der Regel wird die erfindungsgemäße Rückspaltung im Beisein einer Gesamtmenge an sauren Spaltkatalysatoren von, bezogen auf die Menge der zu spaltenden Oxyester, 1 bis 50 Gew.-%, häufig 1 bis 40 Gew.-% oder 5 bis 20 Gew.-% erfolgen.

Ferner kann die erfindungsgemäße Rückspaltung gemäß der Lehre der DE-A 19 547 459 bzw. der DE-A 19 547 485 im zusätzlichen Beisein von monomerer und/oder oligomerer (Meth)acrylsäure durchgeführt werden. Bezogen auf die Menge an zu spaltenden Oxyestern kann die Menge an derartiger monomerer und/oder oligomerer (Meth)acrylsäure bis zu 50 Gew.-% und mehr betragen. Häufig wird vorgenannte Menge an monomerer und/oder oligomerer (Meth)acrylsäure 5 bis 50 Gew.-% oder 10 bis 40 Gew.-% oder 20 bis 35 Gew.-% betragen.

Normalerweise erfolgt der Zusatz der monomeren und/oder oligomeren (Meth)acrylsäure zum der Spaltung zu unterwerfenden Sumpfprodukt in an sich bekannter, mittels Polymerisationsinhibitoren stabilisierter Form. Als oligomere (Meth)acrylsäure kann dabei in besonders einfacher Weise der bei der destillativen Reinigung von Roh-(Meth)acrylsäure anfallende Sumpf eingesetzt werden, der hauptsächlich Verbindungen der allgemeinen Formel (III) enthält (siehe z.B. DE-A 22 35 326). Ein Beisein von monomerer und/oder oligomerer (Meth)acrylsäure bei der erfindungsgemäßen Rückspaltung bedingt vor allem eine reduzierte Ether- und Olefin-Nebenproduktbildung.

Zusätzlich kann, gemäß der Lehre der DE-A 19 701 737, die erfindungsgemäße Rückspaltung des Sumpfproduktes im Beisein von Wasser vorgenommen werden. Bezogen auf die Menge der zu spaltenden Oxyester beträgt vorgenannte Wassermenge in der Regel 0,1 bis 20 Gew.-%, häufig 1 bis 10 Gew.-%.

Das zu spaltende Sumpfprodukt und die erfindungsgemäß zu verwendenden Verbindungen (IV) sowie die gegebenenfalls ebenfalls zuzusetzenden sonstigen sauren Spaltkatalysatoren sowie monomere und/oder oligomere (Meth)acrylsäure und gegebenenfalls Wasser können dem zu spaltenden Sumpfprodukt bereits vor dessen Überführung in den Spaltreaktor zugegeben werden. Sie können dem Spaltreaktor aber auch getrennt zugeführt werden. Bei einem Teil oder der Gesamtmenge der erfindungsgemäß erforderlichen sauren Spaltkatalysatoren kann es sich auch um die sauren Veresterungskatalysatoren handeln. Gemäß einer vorteilhaften Ausbildung der Erfindung wird die erfindungsgemäße Rückspaltung im Beisein von molekularem Sauerstoff durchgeführt.

Besonders günstig ist, wenn durch das zu spaltende Gemisch im Rahmen des erfindungsgemäßen Verfahrens als Schleppmittel für die Spaltprodukte ein Strippgas, das vorzugsweise molekularen Sauerstoff enthält, geführt wird. Zweckmäßigerweise werden als Strippgas Luft oder Gemische von Luft mit Inertgas (z.B. Stickstoff) verwendet.

Die erfindungsgemäß anzuwendende Spalttemperatur beträgt in der Regel 140 bis 260°C, häufig 180 bis 230°C. Vorzugsweise wird die erfindungsgemäße Rückspaltung drucklos bzw. bei reduziertem Druck (< 1 bar), in typischer Weise 500 bis 700 mbar, häufig 40 bis 300 mbar, durchgeführt (so daß die Spaltprodukte unmittelbar abdampfen).

Wird während der Rückspaltung durch das Spaltgemisch ein Strippgas geführt, beträgt dessen Menge im Normalfall 1 - 100 l/h x 1. Im Regelfall wird die Rückspaltung Reaktionsdauern von 1 bis 15 h erfordern. Der Umsatz der Rückspaltung beträgt im Normalfall ≥ 90 Gew.-%.

Zur Durchführung der erfindungsgemäßen Rückspaltung kann z.B. ein einfacher beheizbarer Rührreaktor mit Doppelwandheizung oder Heizschlange oder auch ein Zwangsumlaufverdampfer, beispielsweise ein Fallfilmverdampfer oder Flashverdampfer, gekoppelt mit einem Verweilzeitbehälter, verwendet werden. Zur besseren Trennung der Spaltprodukte vom Sumpfprodukt ist gegebenenfalls eine auf die Spaltapparatur aufgesetzte Rektifikationsvorrichtung, z.B. eine Füllkörper-, Packungs- oder Bodenkolonne, zweckmäßig. Diese Rektifikationsvorrichtung wird in der Regel mit Polymerisationsinhibitoren (z.B. Phenothiazin, Hydrochinonmonomethylether, Hydrochinon etc.) stabilisiert betrieben. Selbstredend ist auch das rückzuspaltende Sumpfprodukt von der Veresterung herrührend mittels Polymerisationsinhibitoren polymerisationsstabilisiert.

Die Reaktionsführung der erfindungsgemäßen Rückspaltung läuft z.B. in der Weise, daß das zu spaltende Sumpfprodukt kontinuierlich aus der destillativen Aufarbeitung des Veresterungsgemisches ausgeschleust und mit dem erfindungsgemäß zu verwendenden Spaltkatalysator, gegebenenfalls monomerer und/oder oligomerer (Meth)acrylsäure sowie gegebenenfalls Wasser dem Spaltreaktor zugeführt wird. Die Reaktionsführung kann aber auch diskontinuierlich, d.h. batchweise, durchgeführt werden. Auch eine halbkontinuierliche Reaktionsführung ist möglich, bei der das zu spaltende Sumpfprodukt sowie die diesem gegebenenfalls zuzugebenden Zusätze kontinuierlich dem Spaltreaktor, der den sauren Spaltkatalysator enthält, zugeführt und das bei der Spaltung anfallende Sumpfprodukt erst nach Beendigung der Spaltung aus dem Spaltreaktor batchweise entfernt wird.

Die bei der erfindungsgemäßen Rückspaltung entstehenden Spaltprodukte (Alkanol, Alkyl(meth)acrylat und (Meth)acrylsäure) werden üblicherweise kontinuierlich dampfförmig abgetrennt und können, im Unterschied zum verfahren der US-A 5 734 075, ohne Zwischenreinigung unmittelbar in die Veresterung zurückgeführt werden. Selbstverständlich könnte die Rückführung aber auch gemäß der US-A 5 734 075 vorgenommen werden.

Wird die Veresterung so durchgeführt, daß das bei der Veresterung gebildete Wasser über eine dem Veresterungsreaktor aufgesetzte Rektifikationskolonne kontinuierlich abgetrennt wird, erfolgt die Rückführung der Spaltprodukte in die Veresterung vorzugsweise über diese Rektifikationskolonne (zweckmäßig wird in die untere Hälfte der Rektifikationskolonne rückgeführt).

Selbstredend kann das erfindungsgemäße Rückspaltungsverfahren auch mehrstufig (z.B. in einer Kaskade, z.B. wie in der CN-A 1063678) durchgeführt werden.

Vorzugsweise wird die erfindungsgemäße Rückspaltung zweistufig durchgeführt, wobei in der ersten Spaltstufe der Gehalt an sauren Spaltkatalysatoren, bezogen auf die enthaltene Menge an zu spaltenden Oxyestern, in der Regel auf 1 bis 20 Gew.-%, und in der zweiten Stufe auf 5 bis 40 Gew.-% eingestellt wird. Die Verweilzeit in den einzelnen Stufen kann dabei gleich oder verschieden sein. Vorzugsweise nimmt sie von der ersten zur letzten Stufe hin zu. Bei einer zweistufigen Durchführung beträgt die Verweilzeit des Spaltgemisches in der ersten Stufe zweckmäßigerweise 1 bis 15 h und in der zweiten Stufe 10 bis 40 h.

Ferner nimmt die Rückspalttemperatur bei einer mehrstufigen Ausführweise vorzugsweise zur letzten Stufe hin zu. Im zweistufigen Fall beträgt die Spalttemperatur in der ersten Stufe zweckmäßigerweise 160 bis 200°C und in der zweiten Stufe 180 bis 220°C.

Der Vorteil der vorstehend beschriebenen stufenförmigen Spaltung besteht darin, daß das bei der Veresterung anfallende Sumpfprodukt in der Regel noch signifikante Mengen des Zielesters enthält, die bei den hohen Spalttemperaturen besonders (radikalisch) polymerisationsanfällig und auch gegen die sauren Spaltkatalysatoren nicht inert sind. Bei vergleichsweise milden Spaltbedingungen in der ersten Stufe können diese Zielesteranteile im wesentlichen unverändert mit den entstehenden Spaltprodukten schonend abgetrennt werden, bevor die Rückspaltung in den nachfolgenden Stufen unter Verschärfung der Spaltungsbedingungen vervollständigt werden kann. Bei einer kontinuierlichen Durchführung einer mehrstufigen Spaltung kann der Druck in aufeinanderfolgenden Stufen gleich oder verschieden sein. Im Fall eines konstanten Druckes kann der Transport von der einen zur anderen Stufe in einfacher Weise durch standgeregelten Überlauf erfolgen. Bei unterschiedlichen Drucken empfiehlt sich ein gepumpter Gemischtransport.

Bei einer mehrstufigen Rückspaltung ist es ferner von Vorteil, wenn der Rückstand der letzten Spaltungsstufe wenigstens teilweise (zweckmäßig 10 bis 80 % seines Gewichtes) in die erste Spaltstufe rückgeführt wird.

Auch können den einzelnen Stufen verschiedene Mengen an monomerer und/oder oligomerer (Meth)acrylsäure, an Wasser und an sauren Spaltkatalysatoren zugefügt werden.

Die Anwendbarkeit des erfindungsgemäßen Spaltverfahrens ist nicht auf eine spezielle Natur des Veresterungsprozesses, als dessen Nebenprodukte die Oxyester, also die Additionsverbindungen I und II, anfallen, beschränkt. In der Regel werden die Ester nach den üblichen Verfahren hergestellt (s. Ullmann's Encyclopedia of Industrial Chemistry, Vol. A1, 5th Ed., VCH, S. 167ff). Selbstverständlich kommen auch mittels Ionentauschern katalysierte Veresterungsprozesse in Betracht.

Ein typisches Beispiel für die Bedingungen, unter denen die der Spaltung der Oxyester vorausgehende Veresterung stattfinden kann, lassen sich kurz wie folgt darstellen:
- Alkohol:(Meth)acrylsäure 1 :: 0,7 - 1,2 (molar)
- Katalysator:: Schwefelsäure oder Sulfonsäuren (z.B. p-Toluolsulfonsäure)
- Katalysatormenge:: 0,1 - 10 Gew.-% (vorzugsweise 0,5-5 Gew.-%) bzgl. Einsatzstoffe
- Stabilisierung:: 200 - 2000 ppm Phenothiazin (bezogen auf das Gewicht der Einsatzstoffe)
- Reaktionstemperatur:: 80 - 160°C, vorzugsweise 90 - 130°C
- Reaktionszeit:: 1 - 10 Std., vorzugsweise 1 - 6 Std.

Gegebenenfalls wird ein Schleppmittel (z.B. Cyclohexan oder Toluol) zur Entfernung des Veresterungswassers eingesetzt. Die Veresterung kann drucklos, mit Überdruck oder Unterdruck, sowohl kontinuierlich als auch diskontinuierlich durchgeführt werden.

Die zur Veresterung eingesetzte Acrylsäure kann z.B. durch katalytische Gasphasenoxidation von Propen und/oder Acrolein oder Propan erzeugt worden sein und in typischer Weise 0,05 bis 0,5 Gew.-% Aldehyde, 0,1 bis 5 Gew.-% Essigsäure, 0,05 bis 2 Gew.-% Maleinsäure/-anhydrid, 0,1 bis 5 Gew.-% Wasser, 0,1 bis 1 Gew.-% an Polymerisationsinhibitoren (z.B. Phenothiazin), 0,1 bis 5 Gew.-% oligomere Acrylsäure und 80 bis 99 Gew.-% Acrylsäure enthalten.

Bei der säurekatalysierten Veresterung von Acrylsäure mit Alkanolen hat das nach der Abtrennung des sauren Veresterungskatalysators, der nicht umgesetzten Ausgangsstoffe und des Acrylesters resultierende Sumpfprodukt in der Regel folgende Zusammensetzung:

| | |
|---|---|
| 1 - 20 Gew.-% | Acrylester |
| 40 - 80 Gew.-% | Alkoxypropionate (s. Formel I) |
| 5 - 30 Gew.-% | Acyloxypropionate (s. Formel II) |
| Rest: | hauptsächlich Stabilisatoren (Phenothiazin) und Polymerisate, |
| 0,1 bis 2 Gew.-% | Maleinsäureester können ebenfalls enthalten sein. |

Weitere Einzelheiten und Vorteile des erfindungsgemäßen Verfahrens können den im folgenden beschriebenen Ausführungsbeispielen entnommen werden.

Der Vorteil der erfindungsgemäßen Verfahrensweise beruht darin, daß der nach der Rückspaltung und Abtrennung der Spaltprodukte verbleibende Rückstand in der Regel pumpfähige Konsistenz aufweist und allenfalls eine minimale Feststoffracht enthält.

### Beispiele und Vergleichsbeispiele

### Beispiel 1

Ein aus Glas bestehender erster Umlaufreaktor (Volumen 1 l), beheizt mit einer Heizkerze, wurde mit 500 g eines vom sauren Veresterungskatalysator befreiten, oxyesterhaltigen Sumpfprodukt der Butylacrylatherstellung gefüllt (im folgenden steht Butyl generell für n-Butyl). Das Sumpfprodukt enthielt 15 Gew.-% Butylacrylat, 60,5 Gew.-% Butoxyester I (R = C₄H₉) und 17 Gew.-% Acyloxyester II (R = C₄H₉). Der ·Rest bestand aus (radikalischen) Polymerisaten, Oligomerisaten und Polymerisationsinhibitor (Phenothiazin).

Anschließend wurden dem Umlaufreaktor 60 g Dodecylbenzolsulfonsäure und 95 g Acrylsäure (bezogen auf das Gewicht der Acrylsäure mit 300 ppm Phenothiazin stabilisiert) zugegeben.

Dem so gefüllten Umlaufreaktor wurden standgeregelt je Stunde 180 g des vorstehenden Sumpfproduktes, 40 g wie oben stabilisierte Acrylsäure und 20 g Dodecylbenzolsulfonsäure kontinuierlich zugeführt.

Die Spalttemperatur betrug 175°C, der Druck betrug 700 mbar. Die Spaltprodukte wurden dampfförmig über eine dem Umlaufreaktor aufgesetzte Füllkörperkolonne (50 cm (Füllhöhe) x 2,8 cm (Innendurchmesser), 0,8 cm (Durchmesser) Raschigringe als Spritzschutz abgeführt und kondensiert. Es fielen stündlich 148 g Kondensat an.

Der Spaltrückstand des ersten Umlaufreaktors wurde standgeregelt (Überlauf) einem baugleichen zweiten Umlaufreaktor zugeführt. Letzterem wurden zusätzlich 40 g/h der oben genannten stabilisierten Acrylsäure zugeführt. Die Spalttemperatur im zweiten Umlaufreaktor betrug 200°C. Der Druck betrug ebenfalls 700 mbar. Die Spaltprodukte des zweiten Umlaufreaktors wurden wie beim ersten Umlaufreaktor abgeführt und kondensiert. Es fielen stündlich 92,5 g Kondensat an. Die beiden anfallenden Kondensatströme wurden vereinigt und gaschromatographisch analysiert. Danach enthielt das vereinigte Kondensat (bezogen auf sein Gewicht):

| | |
|---|---|
| 64,8 Gew.-% | Butylacrylat, |
| 4,2 Gew.-% | Butanol, |
| 0,2 Gew.-% | Dibutylether und |
| 1,4 Gew.-% | Butene. |

Damit betrug der Rückspaltungsumsatz, bezogen auf die im Sumpfprodukt enthaltene Menge an Oxyester, 98 Gew.-%. Der Spaltrückstand (Sumpfaustrag des 2. Umlaufreaktors) war pumpfähig und enthielt keinen Feststoff. Auch nach 24-stündigem lagern des Spaltrückstandes bei 25°C schied sich kein Feststoff ab.

### Beispiel 2

Ein aus Glas bestehender Umlaufreaktor (Volumen 1 l), beheizt mit einer Heizkerze, wurde mit 500 g eines vom sauren Veresterungskatalysator befreiten, oxyesterhaltigen Sumpfprodukt der Butylacrylatherstellung aus Beispiel 1 gefüllt. Anschließend wurden dem Umlaufreaktor 60 g Dodecylbenzolsulfonsäure und 95 g Acrylsäure (bezogen auf das Gewicht der Acrylsäure mit 300 ppm Phenothiazin stabilisiert) zugegeben.

Dem so gefüllten Umlaufreaktor wurden standgeregelt je Stunde 95 g des Sumpfproduktes der Butylacrylatherstellung aus Beispiel 1, 20 g wie oben stabilisierte Acrylsäure und 20 g Dodecylbenzolsulfonsäure kontinuierlich zugeführt. Die Spalttemperatur betrug 195°C, der Druck betrug 700 mbar. Die Spaltprodukte wurden wie in Beispiel 1 dampfförmig über eine dem Spaltreaktor aufgesetzte Füllkörperkolonne (50 cm (Füllhöhe) x 2,8 cm (Innendurchmesser), 0,8 cm (Durchmesser) Raschigringe) als Spritzschutz abgeführt und kondensiert. Es fielen stündlich 73 g Kondensat an.

Gemäß gaschromatographischer Analyse enthielt das Kondensat (bezogen auf sein Gewicht):

| | |
|---|---|
| 61,3 Gew.-% | Butylacrylat, |
| 4,0 Gew.-% | Butanol, |
| 0,3 Gew.-% | Dibutylether und |
| 2,1 Gew.-% | Butene. |

Damit betrug der Rückspaltungsumsatz, bezogen auf die im Sumpfprodukt enthaltene Menge an Oxyester, 97 Gew.-%. Der Spaltrückstand (Sumpfaustrag) war pumpfähig und enthielt keinen Feststoff. Auch nach 24-stündigem lagern des Spaltrückstandes bei 25°C schied sich kein Feststoff ab.

### Vergleichsbeispiel 1

Wie Beispiel 1, die Dodecylbenzolsulfonsäure wurde jedoch durch eine äquimolare Menge an p-Toluolsulfonsäure ersetzt.

Das vereinigte Kondensat enthielt

| | |
|---|---|
| 64,2 Gew.-% | Butylacrylat, |
| 4,5 Gew.-% | Butanol, |
| 0,2 Gew.-% | Dibutylether und |
| 1,5 Gew.-% | Butene. |

Damit betrug der Rückspaltungsumsatz, bezogen auf die im Sumpfprodukt enthaltene Menge an Oxyester, 97 Gew.-%. Der Spaltrückstand war zwar pumpfähig, enthielt jedoch ca. 1 Gew.-% an Feststoffen, die sich beim Stehen des Spaltrückstandes bei 25°C teilweise abschieden.

### Vergleichsbeispiel 2

In einem 1 1-Rührreaktor wurde ein Gemisch aus 180 g des Sumpfproduktes der Butylacrylatherstellung aus Beispiel 1 und 120 g mit Phenothiazin stabilisierter oligomerer Acrylsäure (Sumpfprodukt einer roh-Acrylsäuredestillation) bei 700 mbar und 195°C erhitzt. Die entstehenden Spaltprodukte wurden über eine dem Spaltreaktor aufgesetzte Füllkörperkolonne (30 cm (Füllhöhe) x 2,8 cm (Innendurchmesser), 0,8 cm (Durchmesser) Raschigringe) als Spritzschutz abgeführt und kondensiert. Innerhalb von 6,5 Stunden wurden 160 g Kondensat gewonnen.

Das Kondensat enthielt

| | |
|---|---|
| 64,0 Gew.-% | Acrylsäure, |
| 26,8 Gew.-% | Butylacrylat, |
| 0,8 Gew.-% | Butanol und |
| 6,0 Gew.-% | Butoxypropionsäurebutylester. |

Damit betrug der Rückspaltungsumsatz, bezogen auf die im Sumpfprodukt enthaltene Menge an Oxyester, < 10 Gew.-%.

## Patentansprüche

1. Verfahren zum Verestern von (Meth)acrylsäure mit einem Alkanol in Gegenwart eines Veresterungskatalysators, bei dem man vom Reaktionsgemisch nicht umgesetzte Ausgangsverbindungen und gebildeten (Meth)acrylsäureester unter Verbleib eines Oxyester enthaltenden Sumpfproduktes destillativ entfernt, das Sumpfprodukt abtrennt und nach der Abtrennung des Sumpfproduktes die in selbigem enthaltenen Oxyester im Beisein wenigstens eines Säurekatalysators, wobei als wenigstens ein Säurekatalysator zur Spaltung der Oxyester wenigstens eine aromatische Sulfonsäure der allgemeinen Formel IV, 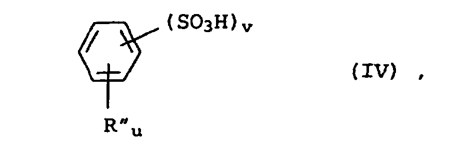 mit
R" = unabhängig voneinander ein sechs bis zwanzig C-Atome aufweisender Alkylrest,
u = eine ganze Zahl von 1 bis 3,
v = 1 oder 2,
mitverwendet wird, durch Einwirkung erhöhter Temperatur spaltet, **dadurch gekennzeichnet, daß** der Anteil der aromatischen Sulfonsäuren der allgemeinen Formel (IV), bezogen auf die Gesamtmenge der zur Spaltung der Oxyester eingesetzten Säurekatalysatoren, wenigstens 50 mol-% beträgt, und die Spaltung stufenförmig durchgeführt wird.

2. Verfahren nach Anspruch 1, **dadurch gekennzeichnet, daß** der Anteil der aromatischen Sulfonsäuren der allgemeinen Formel (IV), bezogen auf die Gesamtmenge der zur Spaltung der Oxyester eingesetzten Säurekatalysatoren, wenigstens 75 mol-% beträgt.

3. Verfahren nach Anspruch 1, **dadurch gekennzeichnet, daß** der Anteil der aromatischen Sulfonsäuren der allgemeinen Formel (IV), bezogen auf die Gesamtmenge der zur Spaltung der Oxyester eingesetzten Säurekatalysatoren, 100 mol-% beträgt.

4. Verfahren nach einem der Ansprüche 1 bis 3, **dadurch gekennzeichnet, daß** die Gesamtmenge an sauren Spaltkatalysatoren, bezogen auf die Menge der zu spaltenden Oxyester, 1 bis 50 Gew.-% beträgt.

5. Verfahren nach einem der Ansprüche 1 bis 3, **dadurch gekennzeichnet, daß** die Gesamtmenge an sauren Spaltkatalysatoren, bezogen auf die Menge der zu spaltenden Oxyester, 5 bis 20 Gew.-% beträgt.

6. Verfahren nach einem der Ansprüche 1 bis 5, **dadurch gekennzeichnet, daß** die Spaltung der im Sumpfprodukt enthaltenen Oxyester im Beisein von, bezogen auf die Menge der zu spaltenden Oxyester, bis zu 50 Gew.-% monomerer und/oder oligomerer (Meth)acrylsäure erfolgt.

7. Verfahren nach einem der Ansprüche 1 bis 5, **dadurch gekennzeichnet, daß** die Spaltung der im Sumpfprodukt enthaltenen Oxyester im Beisein von, bezogen auf die Menge der zu spaltenden Oxyester, 10 bis 40 Gew.-% monomerer und/oder oligomerer (Meth)acrylsäure erfolgt.

8. Verfahren nach einem der Ansprüche 1 bis 7, **dadurch gekennzeichnet, daß** die Spaltung der im Sumpfprodukt enthaltenen Oxyester im Beisein von, bezogen auf die Menge der zu spaltenden Oxyester, 0,1 bis 20 Gew.-% Wasser erfolgt.

9. Verfahren nach einem der Ansprüche 1 bis 7, **dadurch gekennzeichnet, daß** die Spaltung der im Sumpfprodukt enthaltenden Oxyester im Beisein von, bezogen auf die Menge der zu spaltenden Oxyester, 1 bis 10 Gew.-% Wasser erfolgt.

10. Verfahren nach einem der Ansprüche 1 bis 9, **dadurch gekennzeichnet, daß** als wenigstens eine aromatische Sulfonsäure der allgemeinen Formel (IV) Dodecylbenzolsulfonsäure mitverwendet wird.

11. Verfahren nach einem der Ansprüche 1 bis 10, **dadurch gekennzeichnet, daß** die Spalttemperatur 140 bis 260°C beträgt.

12. Verfahren nach einem der Ansprüche 1 bis 10, **dadurch gekennzeichnet, daß** die Spalttemperatur 180 bis 230°C beträgt.

13. Verfahren nach einem der Ansprüche 1 bis 12, **dadurch gekennzeichnet, daß** die Spaltung bei einem Druck von 500 mbar bis 1 bar durchgeführt wird.

14. Verfahren nach einem der Ansprüche 1 bis 13, **dadurch gekennzeichnet, daß** ein Strippgas durch das Sumpfprodukt geführt wird.

15. Verfahren nach einem der Ansprüche 1 bis 13, **dadurch gekennzeichnet, daß** als Strippgas ein Sauerstoff enthaltendes Gas verwendet wird.

16. Verfahren nach einem der Ansprüche 1 bis 15, **dadurch gekennzeichnet, daß** die anfallenden Spaltprodukte unmittelbar in die Veresterung rückgeführt werden.

17. Verfahren nach einem der Ansprüche 1 bis 16, **dadurch gekennzeichnet, daß** das Alkanol ein C₁- bis C₈-Alkanol ist.

18. Verfahren nach einem der Ansprüche 1 bis 17, **dadurch gekennzeichnet, daß** das Alkanol n-Butanol oder 2-Ethylhexanol ist.

19. Verfahren nach einem der Ansprüche 1 bis 18, **dadurch gekennzeichnet, daß** die Spaltung zweistufig durchgeführt wird.

20. Verfahren nach Anspruch 19, **dadurch gekennzeichnet, daß** in der ersten Spaltstufe der Gehalt an sauren Spaltkatalysatoren, bezogen auf die enthaltene Menge an zu spaltenden Oxyestern, 1 bis 20 Gew.-% und in der zweiten Stufe auf 5 bis 40 Gew.-% beträgt

21. Verfahren einem der Ansprüche 19 oder 20, **dadurch gekennzeichnet, daß** die Spalttemperatur in der ersten Stufe 160 bis 200°C und in der zweiten Stufe 180 bis 220°C beträgt.

22. Verfahren nach einem der Ansprüche 19 bis 21, **dadurch gekennzeichnet, daß** die Verweilzeit in der ersten Spaltstufe 1 bis 15 h und in der zweiten Stufe 10 bis 40 h beträgt.

23. Verfahren nach einem der Ansprüche 1 bis 22, **dadurch gekennzeichnet, daß** der Rückstand der letzten Spaltstufe wenigstens teilweise in die erste Spaltstufe rückgeführt wird.

## Claims

1. A process for esterifying (meth)acrylic acid with an alkanol in the presence of an esterification catalyst, in which unconverted starting compounds and (meth)acrylic ester formed are removed from the reaction mixture by distillation, leaving behind an oxyester-containing bottom product, the bottom product is separated off and the oxyesters contained therein are thereafter cleaved by the action of elevated temperature in the presence of at least one acid catalyst, at least one aromatic sulfonic acid of the formula IV, 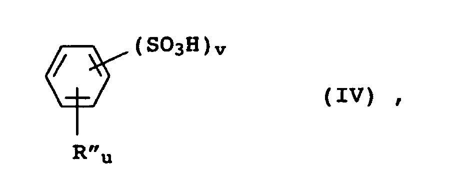 where
R" independently of one another are each alkyl of six to twenty carbon atoms,
u is an integer from 1 to 3, and
v is 1 or 2,
being present as the at least one acid catalyst for cleaving the oxyesters, wherein the amount of the aromatic sulfonic acids of the formula (IV) is at least 50 mol%, based on the total amount of the acid catalysts used for the cleavage of the oxyesters and the cleavage is carried out stepwise.

2. A process as claimed in claim 1, wherein the amount of the aromatic sulfonic acids of the formula (IV) is at least 75 mol%, based on the total amount of the acid catalysts used for the cleavage of the oxyesters.

3. A process as claimed in claim 1, wherein the amount of the aromatic sulfonic acids of the formula (IV) is 100 mol%, based on the total amount of the acid catalysts used for the cleavage of the oxyesters.

4. A process as claimed in any of claims 1 to 3, wherein the total amount of acidic cleavage catalysts is from 1 to 50% by weight, based on the amount of the oxyesters to be cleaved.

5. A process as claimed in any of claims 1 to 3, wherein the total amount of acidic cleavage catalysts is from 5 to 20% by weight, based on the amount of the oxyesters to be cleaved.

6. A process as claimed in any of claims 1 to 5, wherein the cleavage of the oxyesters present in the bottom product is effected in the presence of up to 50% by weight, based on the amount of the oxyesters to be cleaved, of monomeric and/or oligomeric (meth)acrylic acid.

7. A process as claimed in any of claims 1 to 5, wherein the cleavage of the oxyesters present in the bottom product is effected in the presence of from 10 to 40% by weight, based on the amount of the oxyesters to be cleaved, of monomeric and/or oligomeric (meth)acrylic acid.

8. A process as claimed in any of claims 1 to 7, wherein the cleavage of the oxyesters present in the bottom product is effected in the presence of from 0.1 to 20% by weight, based on the amount of the oxyesters to be cleaved, of water.

9. A process as claimed in any of claims 1 to 7, wherein the cleavage of the oxyesters present in the bottom product is effected in the presence of from 1 to 10% by weight, based on the amount of the oxyesters to be cleaved, of water.

10. A process as claimed in any of claims 1 to 9, wherein dodecylbenzenesulfonic acid is present as at least one aromatic sulfonic acid of the formula (IV).

11. A process as claimed in any of claims 1 to 10, wherein the cleavage temperature is from 140 to 260°C.

12. A process as claimed in any of claims 1 to 10, wherein the cleavage temperature is from 180 to 230°C.

13. A process as claimed in any of claims 1 to 12, wherein the cleavage is carried out at from 500 mbar to 1 bar.

14. A process as claimed in any of claims 1 to 13, wherein a stripping gas is passed through the bottom product.

15. A process as claimed in any of claims 1 to 13, wherein the stripping gas used is an oxygen-containing gas.

16. A process as claimed in any of claims 1 to 15, wherein the cleavage products obtained are immediately recycled to the esterification.

17. A process as claimed in any of claims 1 to 16, wherein the alkanol is a C₁- to C₈-alkanol.

18. A process as claimed in any of claims 1 to 17, wherein the alkanol is n-butanol or 2-ethylhexanol.

19. A process as claimed in any of claims 1 to 18, wherein the cleavage is carried out in two stages.

20. A process as claimed in claim 19, wherein the content of acidic cleavage catalysts is from 1 to 20% by weight in the first cleavage stage and from 5 to 40% by weight in the second stage, based on the contained amount of oxyesters to be cleaved.

21. A process as claimed in either of claims 19 and 20, wherein the cleavage temperature in the first stage is from 160 to 200°C and that in the second stage is from 180 to 220°C.

22. A process as claimed in any of claims 19 to 21, wherein the residence time in the first cleavage stage is from 1 to 15 h and that in the second stage is from 10 to 40 h.

23. A process as claimed in any of claims 1 to 22, wherein the residue of the last cleavage stage is at least partly recycled to the first cleavage stage.

## Revendications

1. Procédé d'estérification d'acide (méth)acrylique avec un alcanol, en présence d'un catalyseur d'estérification, dans lequel, par distillation, on élimine du mélange réactionnel des composés de départ n'ayant pas réagi et des esters d'acide (méth)acrylique formés, avec maintien d'un produit de fond de cuve contenant des oxyesters, on isole le produit de fond de cuve et, après l'isolement du produit de fond de cuve, on coupe les oxyesters contenus dans celui-ci par l'action d'une température élevée, en présence d'au moins un catalyseur acide, au moins un acide sulfonique aromatique de la formule générale IV : 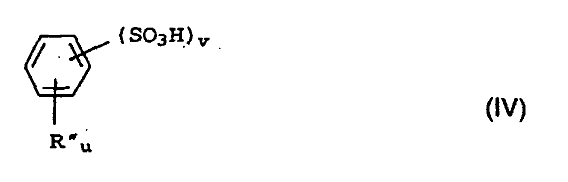 où
R" = indépendamment l'un de l'autre un radical alkyle présentant 6 à 20 atomes de C,
u = un nombre entier de 1 à 3, et
v = 1 ou 2,
étant conjointement utilisé à titre d'au moins un catalyseur acide pour la coupure des oxyesters, **caractérisé en ce que** la fraction des acides sulfoniques aromatiques de la formule (IV), par rapport à la quantité totale des catalyseurs acides mis en oeuvre pour la coupure des oxyesters, est d'au moins 50 moles % et **en ce que** la coupure est effectuée sous une forme étagée.

2. Procédé suivant la revendication 1, **caractérisé en ce que** la fraction des acides sulfoniques aromatiques de la formule générale (IV), par rapport à la quantité totale des catalyseurs acides mis en oeuvre pour la coupure des oxyesters, est d'au moins 75 moles %.

3. Procédé suivant la revendication 1, **caractérisé en ce que** la fraction des acides sulfoniques aromatiques de la formule générale (IV), par rapport à la quantité totale des catalyseurs acides mis en oeuvre pour la coupure des oxyesters, est de 100 moles %.

4. Procédé suivant l'une des revendications 1 à 3, **caractérisé en ce que** la quantité totale de catalyseurs de coupure acides, par rapport à la quantité des oxyesters à couper, est de 1 à 50% en poids.

5. Procédé suivant l'une des revendications 1 à 3, **caractérisé en ce que** la quantité totale de catalyseurs de coupure acides, par rapport à la quantité des oxyesters à couper, est de 5 à 20% en poids.

6. Procédé suivant l'une des revendications 1 à 5, **caractérisé en ce que** la coupure des oxyesters contenus dans le produit de fond de cuve a lieu en présence de jusqu'à 50% en poids d'acide (méth)-acrylique monomère et/ou oligomère, par rapport à la quantité des oxyesters à couper.

7. Procédé suivant l'une des revendications 1 à 5, **caractérisé en ce que** la coupure des oxyesters contenus dans le produit de fond de cuve a lieu en présence de 10 à 40% en poids d'acide (méth)-acrylique monomère et/ou oligomère, par rapport à la quantité des oxyesters à couper.

8. Procédé suivant l'une des revendications 1 à 7, **caractérisé en ce que** la coupure des oxyesters contenus dans le produit de fond de cuve a lieu en présence de 0,1 à 20% en poids d'eau, par rapport à la quantité des oxyesters à couper.

9. Procédé suivant l'une des revendications 1 à 7, **caractérisé en ce que** la coupure des oxyesters contenus dans le produit de fond de cuve a lieu en présence de 1 à 10% en poids d'eau, par rapport à la quantité des oxyesters à couper.

10. Procédé suivant l'une des revendications 1 à 9, **caractérisé en ce que**, comme au moins un acide sulfonique aromatique de la formule générale (IV), on utilise conjointement de l'acide dodécyl-benzènesulfonique.

11. Procédé suivant l'une des revendications 1 à 10, **caractérisé en ce que** la température de coupure est de 140 à 260°C.

12. Procédé suivant l'une des revendications 1 à 10, **caractérisé en ce que** la température de coupure est de 180 à 230°C.

13. Procédé suivant l'une des revendications 1 à 12, **caractérisé en ce que** la coupure est effectuée à une pression de 500 mbars à 1 bar.

14. Procédé suivant l'une des revendications 1 à 13, **caractérisé en ce qu'**un gaz de stripage est guidé au travers du produit de fond de cuve.

15. Procédé suivant l'une des revendications 1 à 13, **caractérisé en ce que**, comme gaz de stripage, on utilise un gaz contenant de l'oxygène.

16. Procédé suivant l'une des revendications 1 à 15, **caractérisé en ce que** les produits de coupure obtenus sont immédiatement recyclés dans l'estérification.

17. Procédé suivant l'une des revendications 1 à 16, **caractérisé en ce que** l'alcanol est un alcanol en C₁-C₈.

18. Procédé suivant l'une des revendications 1 à 17, **caractérisé en ce que** l'alcanol est du n-butanol ou du 2-éthylhexanol.

19. Procédé suivant l'une des revendications 1 à 18, **caractérisé en ce que** la coupure est effectuée en deux étapes.

20. Procédé suivant la revendication 19, **caractérisé en ce que**, dans la première étape de coupure, la teneur en catalyseurs de coupure acides, par rapport à la quantité contenue d'oxyesters à couper, est de 1 à 20% en poids et, dans la deuxième étape, de 5 à 40% en poids.

21. Procédé suivant l'une des revendications 19 et 20, **caractérisé en ce que** la température de coupure dans la première étape est de 160 à 200°C et dans la deuxième étape de 180 à 220°C.

22. Procédé suivant l'une des revendications 19 à 21, **caractérisé en ce que** le temps de séjour dans la première étape de coupure est de 1 à 15 h et dans la deuxième étape de 10 à 40 h.

23. Procédé suivant l'une des revendications 1 à 22, **caractérisé en ce que** le résidu de la dernière étape de coupure est au moins partiellement recyclé dans la première étape de coupure.
